# EUROPEAN PATENT APPLICATION

(11) **EP 1 647 248 A1**
(43) Date of publication of application: **19.04.2006**
(21) Application number: 05108125.5
(22) Date of filing: 05.09.2005
(51) Int. Cl.: A61F 9/007

(54) **Low resistance irrigation system**

(30) Priority: 14.10.2004 US 964964
(71) Applicant: Alcon, Inc., 6331 Hünenberg (CH)
(72) Inventor: Akahoshi, Takayuki, Tokyo, Tokyo 104-0051 (JP)
(74) Representative: Hanna, Peter William Derek

(57) **Abstract**

An irrigation system for a phacoemulsification handpiece (12) is provided comprising a control module (24) capable of controlling an aspiration pump (20), a source of irrigating fluid (16) and a power level supplied to the handpiece. The characteristics of tubing (14) for supplying irrigation fluid from the source of irrigation fluid to the handpiece are selected to affect the irrigation fluid flow resistance in the system. A tubing with an internal diameter of 5mm or a free flow rate of approximately 148 cc/min reduces flow resistance of the system. This provides a more stable intraocular pressure during a phacoemulsification surgical procedure.

## Description

### Background of the Invention

This invention relates generally to the field of cataract surgery and more particularly to a control system for a phacoemulsification handpiece.

The human eye in its simplest terms functions to provide vision by transmitting light through a dear outer portion called the cornea, and focusing the image by way of the lens onto the retina. The quality of the focused image depends on many factors including the size and shape of the eye, and the transparency of the cornea and lens. When age or disease causes the lens to become less transparent, vision deteriorates because of the diminished light which can be transmitted to the retina. This deficiency in the lens of the eye is medically known as a cataract. An accepted treatment for this condition is surgical removal of the lens and replacement of the lens function by an artificial intraocular lens (IOL).

In the United States, the majority of cataractous lenses are removed by a surgical technique called phacoemulsification. During this procedure, a thin phacoemulsification cutting tip is inserted into the diseased lens and vibrated ultrasonically. The vibrating cutting tip liquefies or emulsifies the lens so that the lens may be aspirated out of the eye. The diseased lens, once removed, is replaced by an artificial lens.

A typical ultrasonic surgical device suitable for ophthalmic procedures consists of an ultrasonically driven handpiece, an attached cutting tip, and irrigating sleeve and an electronic control console. The handpiece assembly is attached to the control console by an electric cable and flexible tubings. Through the electric cable, the console varies the power level transmitted by the handpiece to the attached cutting tip and the flexible tubings supply irrigation fluid to and draw aspiration fluid from the eye through the handpiece assembly.

The operative part of the handpiece is a centrally located, hollow resonating bar or horn directly attached to a set of piezoelectric crystals. The crystals supply the required ultrasonic vibration needed to drive both the horn and the attached cutting tip during phacoemulsification and are controlled by the console. The crystal/horn assembly is suspended within the hollow body or shell of the handpiece by flexible mountings. The handpiece body terminates in a reduced diameter portion or nosecone at the body's distal end. The nosecone is externally threaded to accept the irrigation sleeve. Likewise, the horn bore is internally threaded at its distal end to receive the external threads of the cutting tip. The irrigation sleeve also has an internally threaded bore that is screwed onto the external threads of the nosecone. The cutting tip is adjusted so that the tip projects only a predetermined amount past the open end of the irrigating sleeve. Ultrasonic handpieces and cutting tips are more fully described in U.S. Pat. Nos. 3,589,363; 4,223,676; 4,246,902; 4,493,694; 4,515,583; 4,589,415; 4,609,368; 4,869,715; 4,922,902; 4,989,583; 5,154,694 and 5,359,996.

In use, the ends of the cutting tip and irrigating sleeve are inserted into a small incision of predetermined width in the cornea, sclera, or other location. The cutting tip is ultrasonically vibrated along its longitudinal axis within the irrigating sleeve by the crystal driven ultrasonic horn, thereby emulsifying the selected tissue in situ. The hollow bore of the cutting tip communicates with the bore in the horn that in turn communicates with the aspiration line from the handpiece to the console. A reduced pressure or vacuum source in the console draws or aspirates the emulsified tissue from the eye through the open end of the cutting tip, the cutting tip and horn bores and the aspiration line and into a collection device. The aspiration of emulsified tissue is aided by a saline flushing solution or irrigant that is injected into the surgical site through the small annular gap between the inside surface of the irrigating sleeve and the cutting tip.

The preferred surgical technique is to make the incision into the anterior chamber of the eye as small as possible in order to reduce the risk of induced astigmatism. These small incisions result in very tight wounds that squeeze the irrigating sleeve. Such a tight wound construction decreases the stability of the eye, particularly when high aspiration vacuums (above 500 mm Hg) and/or high flows (in excess of 40 cc/min.) are used, because changes in the irrigation flow caused by either changes in the aspiration flow rate or by rapid changes in aspiration vacuum cannot be damped by the inflow of irrigation fluid, which is restricted. Theoretically, increasing the amount of irrigating fluid entering the eye will help to stabilize the intraocular pressure ("IOP"); however, in a clinical setting, the amount of irrigation fluid entering the eye is limited to the amount of fluid aspirated from the eye due to the tight wound construction with minimal leakage from the wound. Also, increasing the flow of irrigating fluid through the eye increases the turbulence in the eye, possibly leading to endothelial cell loss, postoperative inflammation and edema.

Therefore, a need continues to exist for a system that helps to maintain a stable IOP even at high aspiration vacuum levels.

### Brief Summary of the Invention

The present invention improves upon the prior art by providing a surgical irrigation system having reduced irrigation flow resistance, in accordance with claims which follow. The inventor has discovered that change in the intraocular pressure is directly proportional to the irrigation fluid flow resistance in the irrigation system. Therefore, by reducing the irrigation fluid flow resistance in the irrigation system, a more stable IOP can be maintained, even at high aspiration vacuums, without increased fluid flow through the anterior chamber.

Accordingly, one objective of the present invention is to provide a surgical irrigation system having reduced irrigation flow resistance.

Another objective of the present invention is to provide a surgical irrigation system having more stable intraocular pressures.

Another objective of the present invention is to provide a surgical irrigation system that allows for higher aspiration vacuum

Another objective of the present invention is to provide a surgical irrigation system that allows for higher aspiration flow.

These and other advantages and objectives of the present invention will become apparent from the detailed description and claims that follow.

### Brief Description of the Drawings

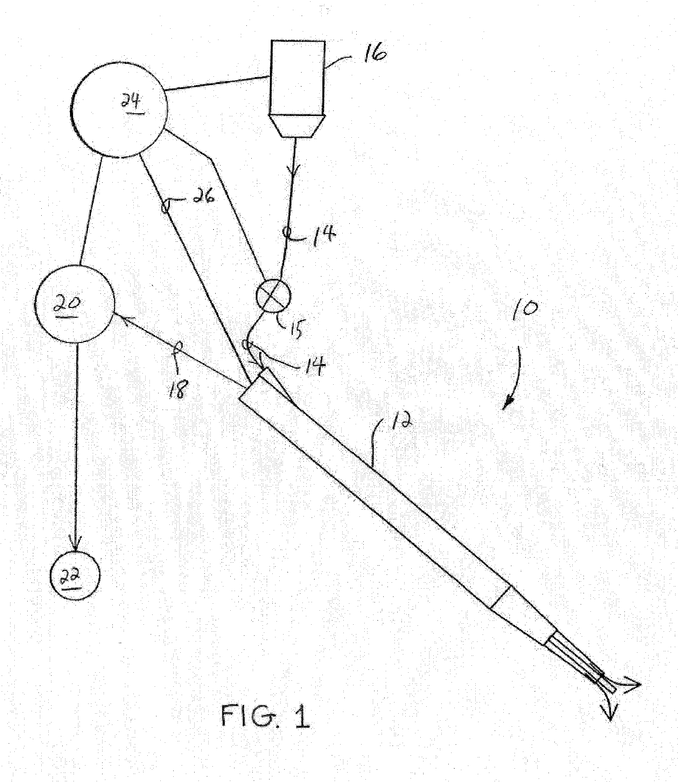

Fig. 1 is a schematic illustration of a phacoemulsification handpiece that may be used with the method and system of the present invention.

### Detailed Description of the Invention

As seen in Figure 1, system 10 that may be used in the method and system of the present invention generally include handpiece 12, which is supplied with irrigating fluid through tubing 14 from source 16. Tubing 14 may contain check valve 15 or some other suitable device for controlling the flow of irrigating fluid in tubing 14. The infusion fluid from source 16 is pressurized either by gravity or by pressurizing source 16. Aspiration line 18 fluidly connects handpiece 12 to pump 20, which aspirates fluid from a surgical site and empties the aspirated fluid into container 22. Handpiece 12 is also electronically connected to control module 24 by cable 26. Control module 24 operates to control aspiration pump 20, infusion source 16, valve 15 and the power supplied to handpiece 12. Suitable control modules are commercially available surgical control consoles such as the LEGACY® SERIES TWENTY THOUSAND ® surgical system or the INFINITI® vision system, both available from Alcon Laboratories, Inc., Fort Worth, Texas.

The inventor has surprisingly discovered that change in the intraocular pressure is directly proportional to the irrigation fluid flow resistance in the irrigation system. Therefore, by reducing the irrigation fluid flow resistance in the irrigation system, a more stable IOP can be maintained, even at high aspiration vacuums, without increased irrigation fluid flow. This reduction in the irrigation fluid flow resistance in the irrigation system is best accomplished by increasing the internal diameter of tubing 14. For example, the inventor has discovered that using irrigation tubing 14 having an internal diameter of 5 mm allows for vastly increased irrigation fluid free flow rates (up to approximately 148 cc/min) indicating greatly reduced resistance to flow in tubing 14. Prior art systems were only capable of much less flow (approximately 100 cc/min.) indicating a much higher resistance to fluid flow.

This description is given for purposes of illustration and explanation. It will be apparent to those skilled in the relevant art that changes and modifications may be made to the invention described above without departing from its scope.

## Claims

1. A method of operating a phacoemulsification handpiece (12) comprising the steps of :
a) providing a control module (24) capable of controlling an aspiration pump (20), a source of irrigating fluid (16) and a power level supplied to the handpiece;
b) supplying an irrigating fluid from the source of irrigation fluid to the handpiece through a tubing (14); and
c) reducing an irrigation fluid flow resistance in the tubing.

2. The method of claim 1, wherein reducing the irrigation flow resistance in the tubing (14) is accomplished without increasing the flow of irrigating fluid through the tubing.

3. The method of claim 1, wherein reducing the irrigation flow resistance in the tubing (14) is accomplished by increasing the internal diameter of the tubing.

4. An irrigation system for a phacoemulsific ation handpiece (12) comprising:
a) a control module (24) capable of controlling an aspiration pump (20), a source of irrigating fluid (16) and a power level supplied to the handpiece;
b) a tubing (14) for supplying irrigation fluid from the source of irrigation fluid to the handpiece, wherein the characteristics of the tubing are selected to affect the irrigation fluid flow resistance in the system.

5. The system of claim 4, wherein the characteristics of the tubing (14) are selected to provide an irrigation fluid free flow rate of up to approximately 148 cc/min.

6. The system of claim 4, wherein tubing (14) having an internal diameter of 5 mm is selected.
